# FASCICULE DE BREVET EUROPEEN

(11) **EP 3 884 265 B1**
(45) Date de publication et mention de la délivrance du brevet: **28.02.2024**
(21) Numéro de dépôt: 19813429.8
(22) Date de dépôt: 20.11.2019
(51) Int. Cl.: G01N 21/17, G01N 29/02, G01N 29/24, B06B 1/02

(54) **CAPTEUR CAPACITIF POUR LA SPECTROSCOPIE PHOTO-ACOUSTIQUE, DISPOSITIF ET PROCÉDÉ METTANT EN OEUVRE UN TEL CAPTEUR**
KAPAZITIVER SENSOR FÜR PHOTOAKUSTISCHE SPEKTROSKOPIE, VORRICHTUNG UND VERFAHREN ZUR VERWENDUNG EINES SOLCHEN SENSORS
CAPACITIVE SENSOR FOR PHOTOACOUSTIC SPECTROSCOPY, DEVICE AND METHOD USING SUCH A SENSOR

(30) Priorité: 21.11.2018 FR 1871680
(43) Date de publication de la demande: 29.09.2021
(73) Titulaire: UNIVERSITE DE MONTPELLIER, 34090 Montpellier (FR); CENTRE NATIONAL DE LA RECHERCHE SCIENTIFIQUE, 75016 Paris (FR)
(72) Inventeur: VICET, Aurore, 34730 Prades-le-Lez (FR); CHAMASSI, Kaim, 34080 Montpellier (FR); BAHRIZ, Michaël, 34080 Montpellier (FR)
(74) Mandataire: IPAZ
(86) Numéro de dépôt international: PCT/EP2019/081911
(87) Numéro de publication internationale: WO 2020/104518

(56) Documents cités:
- WO-A1-2017/186796
- US-A1- 2012 227 498
- US-B1- 6 344 647

## Description

La présente invention concerne un capteur capacitif pour la spectroscopie photo-acoustique, en particulier de gaz. Elle concerne également un dispositif et un procédé de détection, et/ou de mesure, mettant en oeuvre un tel capteur.

Le domaine de l'invention est le domaine des capteurs de spectroscopie photo-acoustique, et en particulier des capteurs de spectroscopie photo-acoustique de gaz.

### Etat de la technique

Dans l'infrarouge, certains gaz présentent des bandes d'absorption de la lumière. Cette caractéristique est utilisée en spectroscopie photo-acoustique pour détecter ces gaz, ou pour mesurer la teneur de ces gaz.

En résumé, un laser modulé à une fréquence donnée éclaire un gaz. Le laser est absorbé par ce gaz et provoque un réchauffement localisé du gaz. L'intensité du laser étant modulée, le réchauffement localisé du gaz est également modulé. La modulation du réchauffement se traduit par la création d'une onde sonore de même fréquence que la fréquence de modulation. Cette onde sonore peut alors être détectée par un capteur de spectroscopie photo-acoustique.

Un premier type de capteur photo-acoustique se présente sous la forme d'une enceinte acoustique résonante équipée d'un microphone. Ce premier type de capteur nécessite l'utilisation d'une enceinte résonante afin d'augmenter le rapport signal sur bruit.

Un deuxième type de capteur photo-acoustique se présente sous la forme d'un capteur à résonateur piézoélectrique. Un tel capteur permet de s'affranchir d'une enceinte acoustique mais reste tout de même complexe, peu compact et peu intégrable.

Par ailleurs, il n'existe actuellement pas de capteur capacitif pour la spectroscopie photo-acoustique. En effet, l'effet d'amortissement, également connu sous le nom de « squeeze film », a toujours constitué un frein pour la conception de capteurs capacitifs pour la spectroscopie photo-acoustique. En effet, la sensibilité de détection dépend de la proximité et de la taille des électrodes capacitives. Or, l'effet d'amortissement augmente avec la proximité et la taille des électrodes, et dégrade la sensibilité de détection.

Un but de la présente invention est de remédier à ces inconvénients.

Un autre but de l'invention est de proposer un capteur capacitif pour la spectroscopie photo-acoustique.

Un autre but de l'invention est de proposer un capteur capacitif pour la spectroscopie photo-acoustique présentant une meilleure sensibilité que les capteurs actuels.

Il est aussi un but de la présente invention de proposer un capteur capacitif pour la spectroscopie photo-acoustique plus compact que les capteurs actuels, et/ou pouvant être intégré.

US 6 344 647 B1 divulgue un spectromètre photo-acoustique miniaturisé pour l'analyse de gaz obtenu par assemblage de quatre éléments intégrés sur semi-conducteur: une source infrarouge modulable électriquement, un filtre optique interférentiel, une microcuve et un microphone capacitif.

### Exposé de l'invention

L'invention permet d'atteindre au moins l'un de ces buts par un capteur pour la spectroscopie photo-acoustique, en particulier de gaz, comprenant :
- un support,
- un résonateur mécanique, fixé audit support, et comprenant :
   - au moins un élément senseur prévu pour être mis en vibration par une onde acoustique, et
   - au moins une première électrode capacitive, mécaniquement couplée audit élément senseur, de sorte à être entraînée par ledit élément senseur lorsqu'il est mis en vibration ; et
- au moins une deuxième électrode capacitive formant avec ladite au moins une première électrode un capteur capacitif.

Ainsi, l'invention propose un capteur capacitif pour la spectroscopie photo-acoustique, en particulier de gaz, dans lequel l'élément senseur de l'onde acoustique est séparé des électrodes capacitives utilisées pour la mesure capacitive. Ainsi, dans le capteur selon l'invention, il est possible de prévoir des architectures différentes d'une part pour la captation de l'onde sonore au niveau de l'élément senseur, et d'autre part pour la détection capacitive au niveau des électrodes capacitives. Il est alors possible d'adopter, au niveau de l'élément senseur, une architecture spécifique limitant, ou diminuant, l'effet d'amortissement « squeeze film » sans pour autant influencer la mesure capacitive. De même, il est possible d'adopter, au niveau des électrodes capacitives, une architecture spécifique augmentant la sensibilité de la détection capacitive, sans influencer l'effet d'amortissement « squeeze film » au niveau de l'élément senseur. Le capteur selon l'invention permet alors une plus grande sensibilité de mesure comparé aux capteurs actuels.

De plus, la technologie de détection capacitive permet d'une part une architecture plus compacte comparée aux capteurs actuels, et d'autre part une réalisation du capteur selon l'invention sous la forme d'un composant intégré.

La technologie capacitive rend possible une réalisation du capteur selon l'invention par une technologie de silicium dopé, sans étape d'implantation ni de dépôt de film métallique, ce qui permet d'augmenter le facteur de qualité du résonateur et de diminuer le coût et la complexité de fabrication.

De manière générale, pour obtenir un capteur capacitif pour la spectroscopie photo-acoustique présentant une bonne sensibilité, la variation de la capacité doit être importante. Pour obtenir une telle variation de capacité, il faut :
- d'une part que la surface sensible à l'onde sonore soit importante ; et
- d'autre part que la distance séparant les condensateurs de mesure capacitive soit la plus petite possible.

Or, satisfaire ces deux conditions revient à rajouter un important amortissement visqueux entre les condensateurs en grande partie à cause d'un mince film d'air entre les condensateurs. C'est cette contradiction qui dissuade l'homme du métier d'utiliser un capteur capacitif pour la spectroscopie photo-acoustique.

Comme expliqué plus haut, la présente invention permet d'éviter cette contradiction en séparant, dans le résonateur, la partie utilisée pour capter l'onde acoustique, à savoir l'élément senseur, de la partie utilisée pour la mesure capacitive, à savoir la première électrode capacitive.

Suivant un mode de réalisation, le résonateur mécanique, et plus généralement le capteur, définit un plan général. Suivant un exemple de réalisation, l'épaisseur du capteur peut être inférieure ou égal à 400µm, voire 600µm.

Avantageusement, le résonateur mécanique peut être agencé pour être mis en mouvement par l'onde sonore dans une direction verticale au plan général du capteur, et/ou du résonateur.

Ainsi, l'amplitude de déplacement du résonateur, et en particulier de l'élément senseur, est augmentée.

Suivant un mode de réalisation particulièrement préféré, au moins une, en particulier chaque, première électrode capacitive est décalée/déportée par rapport à l'élément senseur dans une direction perpendiculaire à la direction de déplacement de l'élément senseur, et/ou dans une direction se trouvant dans le plan général du capteur, et/ou du résonateur.

L'élément senseur peut se présenter sous toute forme géométrique, telle que par exemple en forme de carré, rond, triangle, etc.

Suivant un exemple de réalisation, l'élément senseur a une surface pleine, ou non ajourée.

Selon l'invention, la, ou chaque, première électrode capacitive est mobile.

La première électrode capacitive peut se présenter sous toute forme géométrique, telle que par exemple en forme de croix, rectangle, etc.

Avantageusement, l'élément senseur peut présenter une surface plus grande comparée à la surface d'au moins une, en particulier de chaque, première électrode capacitive.

Ainsi, la captation de l'onde sonore par l'élément senseur est améliorée, tout en diminuant l'effet d'amortissement au niveau la première électrode.

Avantageusement, le résonateur peut présenter un facteur de qualité supérieure ou égale à 10.

Un tel facteur de qualité permet d'améliorer la sélectivité du capteur selon l'invention.

Suivant un mode de réalisation particulièrement avantageux, le support comporte une partie évidée ou ajourée en regard de l'élément senseur.

Ainsi, le capteur selon l'invention diminue, voire annule, tout effet d'amortissement de l'élément senseur lors de son oscillation mécanique, ce qui augmente l'amplitude de mouvement de l'élément senseur et par conséquent la sensibilité dudit capteur.

La partie ajourée peut être formée par une seule ouverture traversante ou non.

Alternativement, la partie ajourée peut être formée par plusieurs ouvertures traversantes ou non.

Avantageusement, le résonateur peut comprendre plusieurs premières électrodes mécaniquement couplées à l'élément senseur et entrainées par l'élément senseur.

L'utilisation d'un plus grand nombre d'électrode permet d'augmenter la sensibilité de la détection capacitive tout en permettant de personnaliser l'architecture du résonateur et donc du capteur.

Lorsque le résonateur comprend plusieurs premières électrodes, il est important que lesdites électrodes soient synchronisées entre elles pour la mesure capacitive de sorte à maintenir en phase les signaux électriques générés par lesdites électrodes.

En particulier, le résonateur peut comprendre quatre premières électrodes capacitives alignées deux à deux avec l'élément senseur, en particulier suivant deux directions perpendiculaires entre elles, de sorte à former une croix centrée sur l'élément senseur.

Une telle architecture du résonateur permet d'améliorer la détection capacitive par l'utilisation de plusieurs premières électrodes capacitives, tout en préservant l'équilibre mécanique pour le résonateur mécanique.

Préférentiellement, le résonateur peut être fixé au support au niveau d'au moins un noeud de vibration mécanique.

Ainsi, les pertes dues à la fixation du résonateur sur le support sont limitées, voire annulées.

Un noeud de vibration est défini comme étant une zone où les déplacements et de contraintes mécaniques sont nuls, ou proches de zéro.

Pour au moins une première électrode capacitive, il est possible de fixer le résonateur au support au niveau d'une position de fixation qui ne se trouve pas entre l'élément senseur et ladite première électrode capacitive.

Alternativement, pour au moins une première électrode capacitive, le résonateur peut être fixé au support en une position de fixation se trouvant entre l'élément senseur et ladite première électrode capacitive.

Cette position de fixation peut être choisie de sorte que, les mouvements mécaniques de l'élément senseur et de la première électrode capacitive sont :
- soit de même sens ;
- soit de sens opposés, de sorte que lorsque l'élément senseur se déplace dans un sens, ladite première électrode capacitive se déplace dans l'autre sens.

Une telle architecture permet d'utiliser la fixation comme un levier afin d'ajuster le mouvement de la première électrode capacitive.

Plus particulièrement, pour au moins une première électrode capacitive, la position de fixation peut être plus proche de l'élément senseur que de ladite première électrode capacitive.

Ainsi, une telle fixation peut être utilisée comme un levier pour amplifier l'amplitude des mouvements de ladite première électrode de mesure. Ainsi, la précision de la détection capacitive réalisée par cette électrode peut être augmentée.

Avantageusement, le résonateur peut être réalisé d'une seule pièce, en particulier à partir d'un même matériau.

Plus particulièrement, le résonateur peut être réalisé par usinage d'une même couche d'un matériau, telle qu'une couche de Silicium, ou de tout autre matériau électriquement conducteur.

Avantageusement, au moins une première électrode capacitive peut être solidaire de l'élément senseur par au moins un bras de liaison de largeur plus petite que celle de l'élément senseur.

Ainsi, l'effet d'amortissement dû au bras de fixation entre l'élément senseur et la première électrode est diminué.

Suivant une caractéristique particulièrement avantageuse, au moins une première électrode capacitive peut être ajourée ou comporter des trous.

Ainsi, l'air peut passer au travers de ladite première électrode, ce qui diminue l'effet d'amortissement qui peut exister lorsque ladite première électrode capacitive est mise en mouvement.

Suivant un exemple de réalisation préféré, au moins une première électrode peut être formée par plusieurs branches en parallèle, distantes les unes des autres.

Une telle architecture permet de diminuer l'effet d'amortissement. De plus, une telle architecture permet aussi d'avoir une faible résistance série et de garder une bonne sensibilité sur le signal électrique.

Chaque branche peut présenter une largeur négligeable, par exemple d'une dizaine de micromètres.

Suivant un mode de réalisation, la, ou chaque, deuxième électrode capacitive peut être fixe.

Suivant un mode de réalisation, au moins une, en particulier chaque, deuxième électrode peut être disposée sur/dans le support, en regard de la, ou d'une, première électrode capacitive.

Suivant un exemple de réalisation, le capteur selon l'invention peut comporter, pour au moins une première électrode capacitive, une deuxième électrode capacitive individuelle à ladite première électrode capacitive.

Alternativement, ou en plus, le capteur selon l'invention peut comporter une deuxième électrode capacitive commune à plusieurs, et en particulier à toutes, les premières électrodes capacitives.

Suivant une caractéristique avantageuse, le capteur selon l'invention peut être réalisé à partir d'une structure constituée d'un empilement de couches d'un matériau conducteur et de matériau isolant.

La structure peut comprendre deux couches conductrices séparées d'une couche d'isolant sacrificielle.

Dans ce cas, le résonateur peut être réalisé à partir d'une première couche conductrice et la deuxième électrode capacitive peut être constituée, ou réalisée à partir, d'une deuxième couche conductrice.

Par exemple, le matériau conducteur peut être du Silicium et le matériau isolant peut être du SiO₂ et la structure peut être une structure de de silicium sur isolant comportant au moins deux couches de silicium.

Bien entendu, le capteur selon l'invention peut en outre comprendre des contacts ou pistes électriques pour polariser électriquement les première(s) et deuxième(s) électrodes capacitives.

Suivant un exemple de réalisation non limitatif, le capteur peut comprendre un contact électrique au niveau d'au moins une fixation du résonateur au support, pour polariser la ou les premières électrodes avec un potentiel de masse, ou avec une tension différente d'un potentiel de masse.

De plus, comme expliqué plus haut, le résonateur peut être réalisé d'une seule pièce à partir d'un même matériau.

Autrement dit, l'ensemble formé par l'élément senseur et l'au moins une première électrode capacitive est réalisé d'une seule pièce et à partir d'un seul matériau.

Dans ce cas, l'élément senseur forme aussi une électrode capacitive reliée ou connectée, à chaque première électrode capacitive et participe à la mesure capacitive en se polarisant de charges électriques injectée dans chaque première électrode capacitive.

Suivant un autre aspect de la même invention, il est proposé un dispositif de détection, et/ou de mesure, pour la spectroscopie photo-acoustique, en particulier de l'air, comprenant :
- au moins une source lumineuse émettant un rayonnement lumineux modulé ; et
- au moins un capteur selon l'invention.

Le dispositif selon l'invention peut comprendre plusieurs sources lumineuses modulées.

Au moins deux sources lumineuses peuvent être modulées à une même fréquence de modulation, ou à des fréquences différentes.

Le dispositif selon l'invention peut comprendre au moins deux sources lumineuses émettant des rayonnements lumineux de différentes longueurs d'ondes.

Il est ainsi possible, avec un même dispositif de détecter différents gaz, ou de mesurer la quantité de différents gaz, absorbant différentes longueurs d'onde, ou encore de différencier deux gaz de spectres d'absorption très proches.

La fréquence de modulation d'au moins une source lumineuse peut en outre être ajustable.

Pour augmenter la sensibilité de mesure, la ou les sources lumineuses peuvent être focalisées en un point se trouvant en regard de l'élément senseur. Ainsi, l'onde sonore peut être générée en regard de l'élément senseur et sa captation par l'élément senseur peut être améliorée.

Au moins une source lumineuse peut être une source laser émettant un rayonnement laser.

Alternativement, ou en plus, au moins une source lumineuse peut être, ou peut comprendre :
- au moins une diode électroluminescente ;
- au moins une diode électroluminescente à cavité résonante ;
- au moins une RC LED dont le spectre d'émission est beaucoup plus fin qu'une LED infrarouge conventionnelle.

Suivant un autre aspect de la même invention, il est proposé un procédé de détection d'un gaz, et/ou de mesure de la concentration d'un gaz, mettant en oeuvre :
- un capteur selon l'invention ; ou
- un dispositif selon l'invention.

En particulier, pour réaliser une détection ou une mesure d'un gaz, le capteur selon l'invention, ou le dispositif selon l'invention, est plongé dans un environnement contenant ledit gaz.

De manière générale, l'invention peut être utilisée pour toute application nécessitant un capteur de gaz.

Par exemple, l'invention peut être mise en oeuvre pour la détection :
- de méthane (CH₄) avec un laser émettant à 1,6µm, ou à 2,3µm ou encore à 3,3µm ;
- d'éthylène (C₂H₄) avec un laser émettant à 3,25µm.

L'invention peut être utilisée pour la détection :
- pour l'analyse de la composition chimique de gaz pour les applications moteur, par exemple dans le domaine de l'automobile pour les carburants ou les gaz d'échappement ;
- pour la surveillance de composés organiques volatiles, en particulier dans le domaine de l'environnement pour la surveillance des BTEX (Benzène, Toluène, Ethylbenzène, Xylènes) ;
- pour l'analyse de l'air exhalé, en particulier dans le domaine médical pour la prévention et l'aide au diagnostic médical ;
- pour le contrôle de procédé de fabrication dans le domaine de l'industrie ;
- pour les contrôles qualité et la maturation des produits, en particulier dans le domaine de l'agroalimentaire.

### Description des figures et modes de réalisation

D'autres avantages et caractéristiques apparaîtront à l'examen de la description détaillée d'un mode de réalisation nullement limitatif, et des dessins annexés sur lesquels
- les FIGURES 1 et 2 sont des représentations schématiques d'un premier exemple de réalisation non limitatif d'un capteur selon l'invention ;
- les FIGURES 3 et 4 sont des représentations schématiques d'un deuxième exemple de réalisation d'un capteur selon l'invention ;
- la FIGURE 5 est une représentation schématique d'un troisième exemple de réalisation d'un capteur selon l'invention ; et
- la FIGURE 6 est une représentation schématique du dispositif selon l'invention.

Il est bien entendu que les modes de réalisation qui seront décrits dans la suite ne sont nullement limitatifs. On pourra notamment imaginer des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieur. Cette sélection comprend au moins une caractéristique de préférence fonctionnelle sans détails structurels, ou avec seulement une partie des détails structurels si cette partie est uniquement suffisante pour conférer un avantage technique ou pour différencier l'invention par rapport à l'état de la technique antérieur.

Sur les figures les éléments communs à plusieurs figures conservent la même référence.

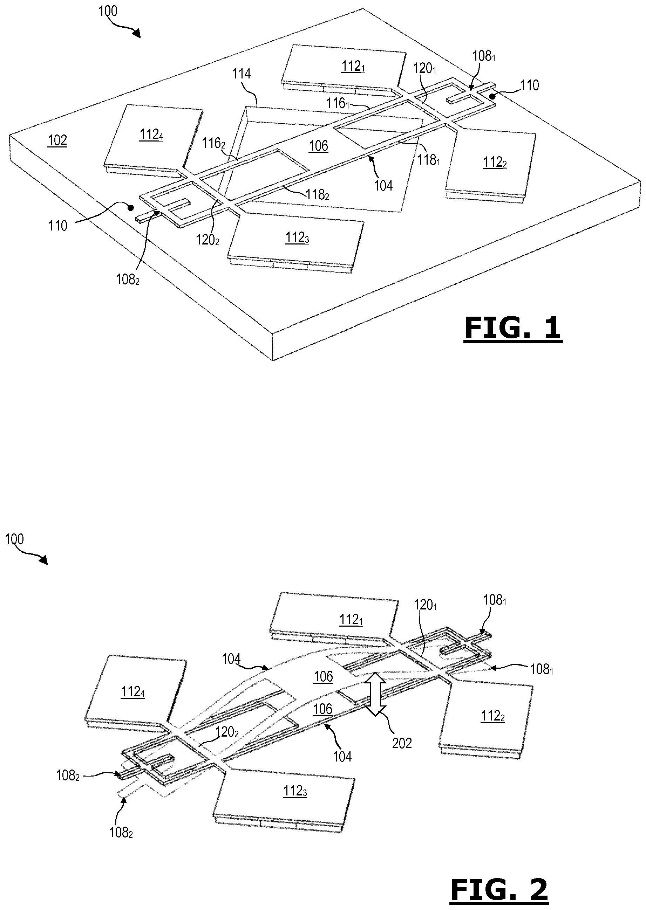

La FIGURE 1 est une représentation schématique d'un premier exemple de réalisation non limitatif d'un capteur selon l'invention.

Le capteur 100, représenté sur la FIGURE 1, comprend un support 102.

Le capteur 100 comprend un résonateur mécanique 104, prévu pour entrer en résonnance mécanique à sa fréquence de résonance, sous l'effet d'une onde sonore.

Le résonateur mécanique 104 comprend un élément senseur 106 sur lequel s'exerce une onde sonore pour mettre en résonance l'ensemble du résonateur 104.

Le résonateur mécanique 104 comprend également au moins une première électrode capacitive décalée et séparée de l'élément senseur, et mécaniquement couplée à l'élément senseur de sorte qu'elle est entraînée par l'élément senseur 106 lorsqu'il est mis en mouvement. Dans l'exemple représenté sur la FIGURE 1, le résonateur mécanique 104 comprend deux premières électrodes 108₁ et 108₂, mécaniquement couplées à l'élément senseur 106, positionnées de part et d'autre de l'élément senseur, et alignées avec l'élément senseur 104.

Le capteur 100 comprend une deuxième électrode capacitive 110, commune aux deux premières électrodes capacitives 108₁ et 108₂, et formant avec chacune desdites premières électrodes capacitives 108₁-108₂ un capteur capacitif. Dans l'exemple représenté sur la FIGURE 1, la deuxième électrode capacitive 110 est formée par l'ensemble du support 102. Pour ce faire, le support 102 est en un matériau électrique conducteur.

Le capteur 100 comprend des blocs de fixation 112₁-112₄ pour rendre le résonateur 104 solidaire du support 102 au niveau de deux noeuds d'oscillation dudit résonateur 104.

Le support 102 comporte au niveau, et en regard, de l'élément senseur 106 une partie 114 entièrement ajourée. Cette partie ajourée 112 permet une circulation d'air de sorte à éviter la formation d'une couche d'air entre l'élément senseur 106 et le support 102, qui pourrait amortir le mouvement de l'élément senseur lors de son oscillation sous l'effet de l'onde sonore.

L'élément senseur 106 est relié à la première électrode capacitive 108₁ par deux bras de liaison 116₁ et 118₁, parallèles, de largeur très faible comparée à celle de l'élément senseur 106 et distante l'une de l'autre. De manière similaire, l'élément senseur 106 est relié à la deuxième électrode capacitive 108₂ par deux bras de liaison 116₂ et 118₂, parallèles, de largeur très faible comparée à celle de l'élément senseur 106 et distante l'une de l'autre.

Ainsi, la liaison entre l'élément senseur 104 et les premières électrodes capacitives 108₁-108₂ ne souffre pas d'un effet d'amortissement lors de l'oscillation mécanique du résonateur 104.

De plus, le résonateur 104 comprend une première branche 120₁, dite de fixation, permettant de fixer le résonateur 104 aux blocs de fixation 112₁-112₂. Cette branche de fixation 120₁ crée une ligne de fixation se trouvant au niveau d'un noeud d'oscillation entre l'élément senseur 106 et la première électrode capacitive 108₁. Cette branche de fixation 120₁ est maintenue mobile, de part et d'autre, dans les blocs 112₁-112₂.

De manière similaire, le résonateur 104 comprend une deuxième branche 120₂, dite de fixation, permettant de fixer le résonateur 104 aux blocs de fixation 112₃-112₄. Cette branche de fixation 120₂ crée une ligne de fixation se trouvant au niveau d'un noeud d'oscillation entre l'élément senseur 106 et la première électrode capacitive 108₂. Cette branche de fixation 120₂ est maintenue mobile, de part et d'autre, dans les blocs 112₃-112₄

Dans le capteur 100 de la FIGURE 1, les premières électrodes capacitives 108₁-108₂ sont identiques.

Dans l'exemple représenté sur la FIGURE 1, chaque première électrode capacitive 108₁-108₂, comporte deux branches, perpendiculaires entre-elles de sorte à former une croix ou un « + ».

Une telle architecture permet de limiter l'effet d'amortissement au niveau de chacune de ces électrodes, tout en rapprochant au maximum chacune des premières électrodes capacitives 108₁-108₂ de la deuxième électrode capacitive 110.

De plus, chaque première électrode capacitive 108₁-108₂ est polarisée à un potentiel électrique non nul par des contacts électriques (non visibles) au niveau des blocs de fixation 112₁-112₄. Le potentiel électrique est propagé dans l'ensemble du résonateur, et en particulier dans les premières électrodes capacitives 108₁-108₂ grâce aux branches de fixation 120₁-120₂ maintenues dans les blocs de fixation 112₁-112₄.

Dans l'exemple représenté, l'élément senseur 106 et les premières électrodes capacitives 108₁-108₂, sont réalisées en une seule pièce/couche. Plus généralement, l'ensemble du résonateur est réalisé en une seule pièce/couche.

Comme visible sur la FIGURE 1, le capteur 100 définit un plan général.

L'élément senseur 106 est prévu pour capter une onde sonore dans la direction perpendiculaire au plan principal du capteur 100. L'élément senseur 106, et plus généralement le résonateur 104, est prévu pour se déformer dans la direction perpendiculaire au plan principal.

Les premières électrodes 108₁-108₂ sont séparées/déportées de l'élément senseur 106 dans le plan principal du capteur, ou du moins dans une direction perpendiculaire à la direction dans laquelle l'élément senseur se déplace sous l'effet d'une onde acoustique.

La FIGURE 2 est une représentation du capteur de la FIGURE 1, sans le support 102.

Sur la FIGURE 2, le résonateur 104 est représenté dans un état au repos, et dans un état de déformation sous l'effet d'une onde sonore.

Comme expliqué plus haut, l'élément senseur, et plus généralement le résonateur est mis en mouvement dans la direction matérialisée par la double flèche 202, perpendiculaire au plan du capteur, qui est également le plan du résonateur 104 lorsqu'il est au repos.

La FIGURE 2 montre clairement l'élément senseur 106 qui entraîne dans son mouvement chacune des premières électrodes capacitives 108₁-108₂. Le sens de mouvement de l'élément senseur 106 est opposé à celui des premières électrodes capacitives 108₁-108₂.

De plus, le résonateur présente un déplacement nul, ou quasi-nul, au niveau des branches de fixation 120₁-120₂ qui correspondent à des noeuds d'oscillation.

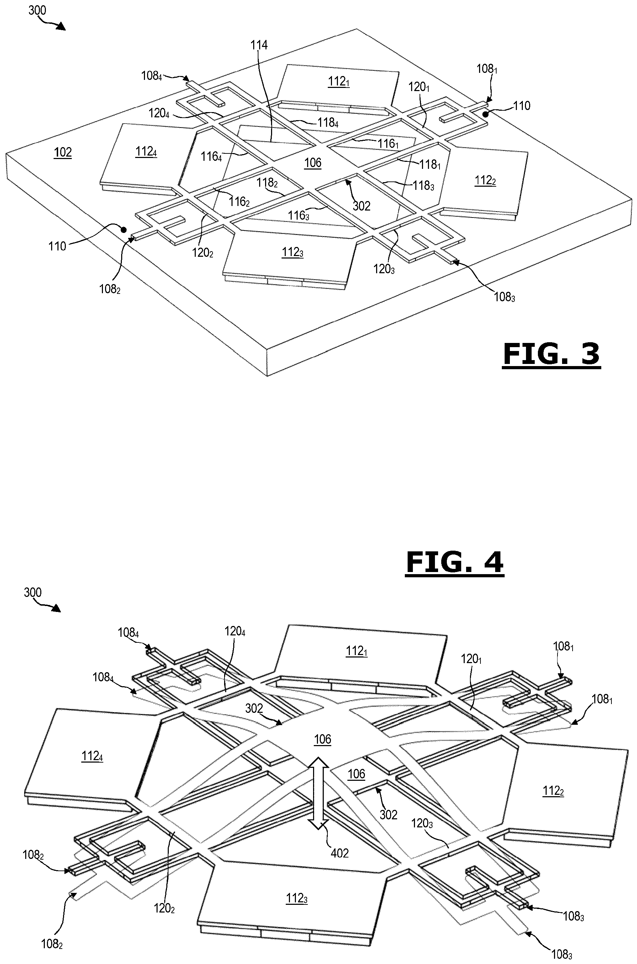

La FIGURE 3 est une représentation schématique d'un deuxième exemple de réalisation non limitatif d'un capteur selon l'invention.

Le capteur 300, représenté sur la FIGURE 3, comprend tous les éléments du capteur 100 de la FIGURE 1.

Le capteur 300 comprend un résonateur 302 qui comprend tous les éléments du résonateur 104 du capteur 100 de la FIGURE 1.

Le résonateur 302 comprend, en plus des éléments du résonateur 104, deux autres premières électrodes 108₃-108₄, positionnées de part et d'autre de l'élément senseur 106.

Les premières électrodes 108₃-108₄ sont alignées avec l'élément senseur 106 dans une direction différente de celle formée par les premières électrodes 108₁-108₂ avec l'élément senseur 106. Ainsi, les premières électrodes 108₁-108₂ et les premières électrodes 108₃-108₄ forment une croix au centre de laquelle se trouve l'élément senseur 106. En particulier les branches de la croix ainsi formée sont perpendiculaires entre elles.

Les premières électrodes capacitives 108₃ et 108₄ sont identiques aux premières électrodes 108₁ et 108₂.

L'élément senseur 106 est relié à la première électrode capacitive 108₃ par deux bras de liaison 116₃ et 118₃, parallèles, de largeur très faible comparée à celle de l'élément senseur 106 et distante l'une de l'autre. De manière similaire, l'élément senseur 106 est relié à la première électrode capacitive 108₄ par deux bras de liaison 116₄ et 118₄, parallèles, de largeur très faible comparée à celle de l'élément senseur 106 et distante l'une de l'autre.

De plus, le résonateur 104 comprend une troisième branche 120₃, dite de fixation, permettant de fixer le résonateur 104 aux blocs de fixation 112₂-112₃. Cette branche de fixation 120₃ crée une ligne de fixation se trouvant au niveau d'un noeud d'oscillation entre l'élément senseur 106 et la première électrode capacitive 108₃. Cette branche de fixation 120₃ est maintenue mobile, de part et d'autre, dans les blocs 112₂-112₃.

De manière similaire, le résonateur 104 comprend une quatrième branche 120₄, dite de fixation, permettant de fixer le résonateur 104 aux blocs de fixation 112₄-112₁. Cette branche de fixation 120₄ crée une ligne de fixation se trouvant au niveau d'un noeud d'oscillation entre l'élément senseur 106 et la première électrode capacitive 108₄. Cette branche de fixation 120₄ est maintenue mobile, de part et d'autre, dans les blocs 112₄-112₁.

Ainsi, le résonateur 302 comprend quatre premières électrodes capacitives 108₁-108₄, identiques.

La FIGURE 4 est une représentation du capteur de la FIGURE 1, sans le support 102.

Sur la FIGURE 4, le résonateur 302 est représenté dans un état au repos, et dans un état de déformation sous l'effet d'une onde sonore.

Comme expliqué plus haut, l'élément senseur 106, et plus généralement le résonateur 302 est mis en mouvement dans la direction matérialisée par la double flèche 402, perpendiculaire au plan du capteur 300, qui est également le plan du résonateur 302 lorsqu'il est au repos.

La FIGURE 4 montre clairement l'élément senseur 106 qui entraîne dans son mouvement chacune des premières électrodes capacitives 108₁-108₄. Le sens de mouvement de l'élément senseur 106 est opposé à celui des premières électrodes capacitives 108₁-108₄.

De plus, le résonateur présente un déplacement nul, ou quasi-nul, au niveau des branches de fixation 120₁-120₄ qui correspondent à des noeuds d'oscillation.

Dans les exemples décrits, le capteur comprend une seule deuxième électrode capacitive formée par le support.

Alternativement, la deuxième électrode capacitive 110 peut être formée par une couche, ou une piste, de matériau conducteur déposée sur le support 102, ou prévue dans l'épaisseur du support 102, ou encore déposée sous le support 102.

Alternativement ou en plus, le capteur 100 peut comprendre plusieurs deuxièmes électrodes capacitives, en particulier une deuxième électrode capacitive individuelle pour chaque première électrode capacitive.

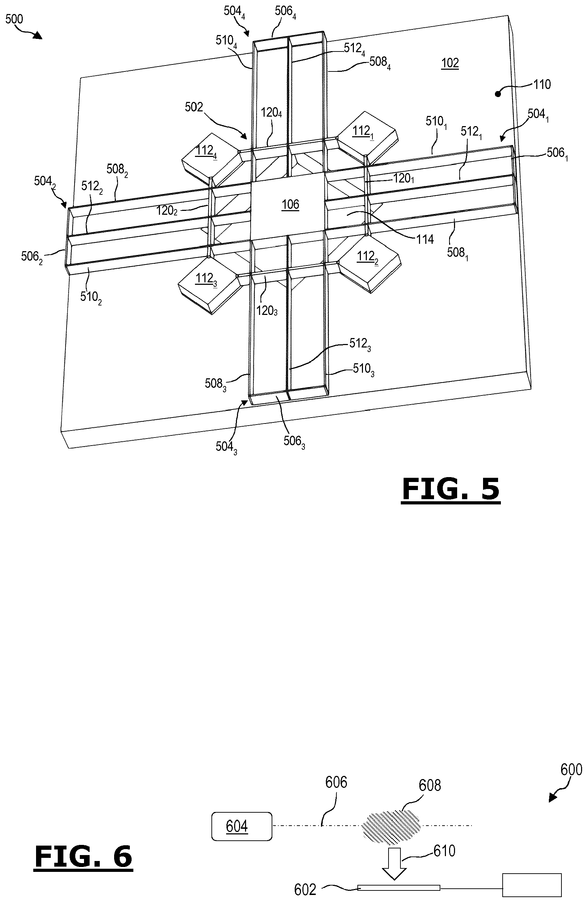

La FIGURE 5 est une représentation schématique d'un troisième exemple de réalisation non limitatif d'un capteur selon l'invention.

Le capteur 500, représenté sur la FIGURE 5, comprend le support 102 faisant office de deuxième électrode capacitive 110, et comportant la partie ajourée 114.

Le capteur 500 comprend également un résonateur 502 comprenant l'élément senseur 106 en regard de la partie ajourée 114. Le résonateur 502 est fixé au support 102 avec les blocs de fixation 112₁-112₄, grâce aux branches de fixation 120₁-120₂ maintenues dans les blocs de fixation 112₁-112₄, et chacune positionnée au niveau d'un noeud de vibration du résonateur 502.

Le résonateur 502 comprend quatre premières électrodes capacitives 504₁-504₄, identiques, de forme différente de celle des électrodes 108₁-108₄.

En particulier, chaque première électrode capacitive 504ᵢ, avec i=1...4, est formée par une branche distale 506ᵢ reliée à l'élément senseur 106 par trois branches 508ᵢ, 510ᵢ et 512ᵢ, parallèle entre-elles, et perpendiculaires à la branche distale 506ᵢ. Par exemple, la première électrode capacitive 504₁ est formée par une branche distale 506₁ reliée à l'élément senseur 106 par trois branches 508₁, 510ᵢ et 512₁, parallèle entre-elles, et perpendiculaires à la branche distale 506ᵢ. La largeur de chacune des branches 506ᵢ, 508ᵢ, 510ᵢ et 512ᵢ est très faible, de l'ordre d'une dizaine de micromètres.

De plus, il est à remarquer que les branches de fixation 120₁-120₂ se trouvent chacun sur un noeud de vibration, en une position plus proche de l'élément senseur que des branches distales 506₁-506₄ faisant partie des premières électrodes capacitives 504₁-504₄. Cette architecture permet de créer un effet de levier amplifiant le mouvement mécanique des premières électrodes capacitives 504₁-504₄. Ainsi, la sensibilité du capteur 500 est améliorée.

La FIGURE 6 est une représentation d'un exemple de réalisation non limitatif d'un dispositif de détection et/ou de mesure selon l'invention.

Le dispositif 600 de la FIGURE 6 comprend un capteur 602 qui peut être l'un quelconque des capteurs 100, 300 ou 500 des FIGURES 1-5.

Le dispositif 600 comprend en outre une source laser 604 émettant un rayonnement laser 606 modulé à une fréquence de modulation donnée et d'une longueur d'onde donnée en direction d'un environnement gazeux 608.

La fréquence de modulation peut être comprise entre 1-100 kHz, et en particulier entre 10-50 kHz

Le rayonnement laser modulé 606 est absorbé par le gaz 608 qui en réponse émet une onde sonore 610 qui est détecté par le capteur 602.

Le dispositif 600 comprend en outre une électronique de détection pour d'une part polariser les électrodes capacitives du capteur 602 et d'autre part mesurer un signal électrique représentatif de la détection capacitive.

Dans l'exemple représenté, le dispositif de détection/mesure comprend une unique source laser modulée.

Alternativement, le dispositif peut comprendre plusieurs sources laser émettant des rayonnements laser modulés, à une même fréquence, mais de longueurs d'onde différentes.

Alternativement, ou en plus, le dispositif peut comprendre une source lumineuse qui n'est pas une source laser. Par exemple, le dispositif peut comprendre au moins une diode électroluminescente, au moins une diode électroluminescente à cavité résonante, et au moins une RC LED dont le spectre d'émission est beaucoup plus fin qu'une LED infrarouge conventionnelle.

Avantageusement, mais sans être limitatif, chaque résonateur mécanique 104, 302 et 502 est réalisé en une seule pièce d'un même matériau. Autrement dit, dans chaque résonateur mécanique :
- l'élément senseur 106,
- les premières électrodes capacitives, et
- les branches de liaison reliant l'élément senseur et chaque première électrode capacitive ;
sont réalisés d'une seule pièce et avec un même matériau. Par conséquent, l'élément senseur et les branches de liaison se comportent également comme un capteur capacitif relié ou connecté à chaque première électrode capacitive.

Bien entendu, l'invention n'est pas limitée aux exemples détaillés cidessus mais des modifications sont possibles dans le cadre des revendications cijointes.

## Revendications

1. Capteur (100;300;500) pour la spectroscopie photo-acoustique comprenant :
- un support (102),
- un résonateur mécanique (104;302;502), fixé audit support (102), et comprenant :
• au moins un élément senseur (106) prévu pour être mis en vibration par une onde acoustique, et
• au moins une première électrode capacitive (108₁-108₄;504₁-504₄) mécaniquement couplée audit élément senseur (106) de sorte à être entraînée par ledit élément senseur (106) lorsqu'il est mis en vibration ; et
- au moins une deuxième électrode capacitive (110) formant avec ladite au moins une première électrode (108₁-108₄;504₁-504₄) un capteur capacitif ;
**caractérisé en ce que** ledit support (102) comporte, en regard dudit élément senseur (106), une partie (114) ajourée formée par une ou plusieurs ouvertures traversantes.

2. Capteur (100;300;500) selon la revendication précédente, **caractérisé en ce que** le résonateur (104;302;502) comprend plusieurs premières électrodes (108₁-108₄;504₁-504₄) mécaniquement couplées à l'élément senseur (106) et entraînées par ledit élément senseur (106).

3. Capteur (300;500) selon l'une quelconque des revendications précédentes, **caractérisé en ce que** le résonateur (302;502) comprend quatre premières électrodes capacitives (108₁-108₄;504₁-504₄) alignées deux à deux avec l'élément senseur (106), de sorte à former une croix centrée sur l'élément senseur (106).

4. Capteur (100;300;500) selon l'une quelconque des revendications précédentes, **caractérisé en ce que** le résonateur (104;302;502) est fixé au support (102) au niveau d'au moins un noeud de vibration mécanique.

5. Capteur (100;300;500) selon l'une quelconque des revendications précédentes, **caractérisé en ce que**, pour au moins une première électrode capacitive (108₁-108₄;504₁-504₄), le résonateur (104;302;502) est fixé au support (102) en une position de fixation se trouvant entre l'élément senseur (106) et ladite première électrode capacitive (108₁-108₄;504₁-504₄).

6. Capteur (500) selon la revendication précédente, **caractérisé en ce que**, pour au moins une première électrode capacitive (504₁-504₄), la position de fixation se trouve plus proche de l'élément senseur (106) que de ladite première électrode capacitive (504₁-504₄).

7. Capteur (100;300;500) selon l'une quelconque des revendications précédentes, **caractérisé en ce que** le résonateur (104;302;502) est réalisé d'une seule pièce.

8. Capteur (100;300;500) selon l'une quelconque des revendications précédentes, **caractérisé en ce que** au moins une première électrode capacitive (108₁-108₄;504₁-504₄) est solidaire de l'élément senseur (106) par au moins un bras de liaison (116₁-116₄,118₁-118₄) de largeur plus petite que celle de l'élément senseur (106).

9. Capteur (500) selon l'une quelconque des revendications précédentes, **caractérisé en ce qu'**au moins une première électrode capacitive (504₁-504₄) est formée par plusieurs branches, de largeur négligeable, distantes les unes des autres.

10. Capteur (100;300;500) selon l'une quelconque des revendications précédentes, **caractérisé en ce que** qu'au moins une, en particulier chaque, deuxième électrode (110) est disposée sur/dans le support (102), en regard de la, ou d'une, première électrode capacitive (108₁-108₄;504₁-504₄).

11. Capteur (100;300;500) selon l'une quelconque des revendications précédentes, **caractérisé en ce qu'**il est réalisé à partir d'une structure constituée d'un empilement de couches d'un matériau conducteur et de matériau isolant.

12. Dispositif (600) de détection, et/ou de mesure, pour la spectroscopie photo-acoustique comprenant :
- au moins une source lumineuse (604) émettant un rayonnement lumineux modulé (606) ; et
- au moins un capteur (602) selon l'une quelconque des revendications précédentes.

13. Dispositif selon la revendication précédente, **caractérisé en ce qu'**il comprend plusieurs sources lumineuses modulées.

14. Dispositif de mesure selon l'une quelconque des revendications 12 ou 13, **caractérisé en ce que** la fréquence de modulation d'au moins une source lumineuse est ajustable.

15. Procédé de détection d'un gaz, et/ou de mesure de la concentration d'un gaz, mettant en oeuvre :
- un capteur (100;300;500) selon l'une quelconque des revendications 1 à 11 ; ou
- un dispositif (600) selon l'une quelconque des revendications 12-14.

## Patentansprüche

1. Sensor (100;300;500) für die photoakustische Spektroskopie, umfassend:
- einen Träger (102),
- einen mechanischen Resonator (104;302;502), der an dem Träger (102) befestigt ist, und umfassend:
• mindestens ein Sensorelement (106), das vorgesehen ist, um durch eine Schallwelle in Schwingung versetzt zu werden, und
• mindestens eine erste kapazitive Elektrode (108₁-108₄;504₁-504₄), die mit dem Sensorelement (106) mechanisch gekoppelt ist, um durch das Sensorelement (106) angetrieben zu werden, wenn es in Schwingung versetzt ist; und
- mindestens eine zweite kapazitive Elektrode (110), die mit der mindestens einen ersten Elektrode (108₁-108₄;504₁-504₄) einen kapazitiven Sensor ausbildet;
**dadurch gekennzeichnet, dass** der Träger (102) gegenüber des Sensorelements (106) einen gelochten Teil (114) aufweist, der durch eine oder mehrere Durchgangsöffnungen ausgebildet ist.

2. Sensor (100;300;500) nach dem vorstehenden Anspruch,
**dadurch gekennzeichnet, dass** der Resonator (104;302;502) mehrere erste Elektroden (108₁-108₄;504₁-504₄) umfasst, die mit dem Sensorelement (106) mechanisch gekoppelt sind und durch das Sensorelement (106) angetrieben werden.

3. Sensor (300;500) nach einem der vorstehenden Ansprüche,
**dadurch gekennzeichnet, dass** der Resonator (302;502) vier erste kapazitive Elektroden (108₁-108₄;504₁-504₄) umfasst, die an dem Sensorelement (106) paarweise ausgerichtet sind, um ein Kreuz auszubilden, das auf dem Sensorelement (106) zentriert ist.

4. Sensor (100;300;500) nach einem der vorstehenden Ansprüche, **dadurch gekennzeichnet, dass** der Resonator (104;302;502) an dem Träger (102) auf Höhe mindestens eines mechanischen Vibrationsknotens befestigt ist.

5. Sensor (100;300;500) nach einem der vorstehenden Ansprüche, **dadurch gekennzeichnet, dass** für mindestens eine erste kapazitive Elektrode (108₁-108₄;504₁-504₄) der Resonator (104;302;502) an dem Träger (102) in einer Befestigungsposition befestigt ist, die sich zwischen dem Sensorelement (106) und der ersten kapazitiven Elektrode (108₁-108₄;504₁-504₄) befindet.

6. Sensor (500) nach dem vorstehenden Anspruch,
**dadurch gekennzeichnet, dass** für mindestens eine erste kapazitive Elektrode (504₁-504₄) sich die Befestigungsposition näher an dem Sensorelement (106) als an der ersten kapazitiven Elektrode (504₁-504₄) befindet.

7. Sensor (100;300;500) nach einem der vorstehenden Ansprüche, **dadurch gekennzeichnet, dass** der Resonator (104;302;502) aus einem einzigen Stück hergestellt ist.

8. Sensor (100;300;500) nach einem der vorstehenden Ansprüche, **dadurch gekennzeichnet, dass** mindestens eine erste kapazitive Elektrode (108₁-108₄;504₁-504₄) mit dem Sensorelement (106) durch mindestens einen Verbindungsarm (116₁-116₄,118₁-118₄) fest verbunden ist, dessen Breite kleiner als die des Sensorelements (106) ist.

9. Sensor (500) nach einem der vorstehenden Ansprüche,
**dadurch gekennzeichnet, dass** mindestens eine erste kapazitive Elektrode (504₁-504₄) durch mehrere Schenkel mit vernachlässigbarer Breite ausgebildet ist, die voneinander beabstandet sind.

10. Sensor (100;300;500) nach einem der vorstehenden Ansprüche, **dadurch gekennzeichnet, dass** mindestens eine, insbesondere jede, zweite Elektrode (110) auf/in dem Träger (102) gegenüber der oder einer ersten kapazitiven Elektrode (108₁-108₄;504₁-504₄) angeordnet ist.

11. Sensor (100;300;500) nach einem der vorstehenden Ansprüche, **dadurch gekennzeichnet, dass** er aus einer Struktur hergestellt ist, die aus einem Stapel von Schichten aus einem leitfähigen Material und Isoliermaterial hergestellt ist.

12. Erfassungs- und/oder Messvorrichtung (600) für die photoakustische Spektroskopie, umfassend:
- mindestens eine Lichtquelle (604), die eine modulierte Lichtstrahlung (606) emittiert; und
- mindestens einen Sensor (602) nach einem der vorstehenden Ansprüche.

13. Vorrichtung nach dem vorstehenden Anspruch,
**dadurch gekennzeichnet, dass** sie mehrere modulierte Lichtquellen umfasst.

14. Messvorrichtung nach einem der Ansprüche 12 oder 13,
**dadurch gekennzeichnet, dass** die Modulationsfrequenz mindestens einer Lichtquelle anpassbar ist.

15. Verfahren zum Erfassen eines Gases und oder zum Messen der Konzentration eines Gases, das implementiert:
- einen Sensor (100;300;500) nach einem der Ansprüche 1 bis 11; oder
- eine Vorrichtung (600) nach einem der Ansprüche 12 bis 14.

## Claims

1. A sensor (100;300;500) for photoacoustic spectroscopy comprising:
- a support (102),
- a mechanical resonator (104;302;502), fastened to said support (102), and comprising:
• at least one sensor element (106) intended to be vibrated by an acoustic wave, and
• at least one first capacitive electrode (108₁-108₄;504₁-504₄), mechanically coupled to said sensor element (106), so as to be moved by said sensor element (106) when it is vibrated; and
- at least one second capacitive electrode (110) forming, with said at least one first electrode (108₁-108₄;504₁-504₄), a capacitive sensor;
**characterized in that** said support (102) has, opposite said sensor element (106), a perforated part (114) formed by one or more through holes.

2. The sensor (100;300;500) according to the preceding claim, **characterized in that** the resonator (104;302;502) comprises several first electrodes (108₁-108₄;504₁-504₄) mechanically coupled to the sensor element (106) and moved by said sensor element (106).

3. The sensor (300;500) according to any one of the preceding claims, **characterized in that** the resonator (302;502) comprises four first capacitive electrodes (108₁-108₄;504₁-504₄) aligned in pairs with the sensor element (106), so as to form a cross centered on the sensor element (106).

4. The sensor (100;300;500) according to any one of the preceding claims, **characterized in that** the resonator (104;302;502) is fastened to the support (102) at the level of at least one mechanical vibration node.

5. The sensor (100;300;500) according to any one of the preceding claims, **characterized in that**, for at least one first capacitive electrode (108₁-108₄;504₁-504₄), the resonator (104;302;502) is fastened to the support (102) at a fastening position located between the sensor element (106) and said first capacitive electrode (108₁-108₄;504₁-504₄).

6. The sensor (500) according to the preceding claim, **characterized in that**, for at least one first capacitive electrode (504₁-504₄), the fastening position is closer to the sensor element (106) than said first capacitive electrode (504₁-504₄).

7. The sensor (100;300;500) according to any one of the preceding claims, **characterized in that** the resonator (104;302;502) is produced in a single piece.

8. The sensor (100;300;500) according to any one of the preceding claims, **characterized in that** at least one first capacitive electrode (108₁-108₄;504₁-504₄) is integral with the sensor element (106) through at least one linking arm (116₁-116₄,118₁-118₄) having a width that is smaller than that of the sensor element (106).

9. The sensor (500) according to any one of the preceding claims, **characterized in that** at least one first capacitive electrode (504₁-504₄) is formed by several branches, having a negligible width, at a distance from one another.

10. The sensor (100;300;500) according to any one of the preceding claims, **characterized in that** at least one, in particular each, second electrode (110) is arranged on/in the support (102), opposite the, or a, first capacitive electrode (108₁-108₄;504₁-504₄).

11. The sensor (100;300;500) according to any one of the preceding claims, **characterized in that** it is produced from a structure constituted by a stack of layers of a conductive material and insulating material.

12. A detection and/or measurement device (600) for photoacoustic spectroscopy comprising:
- at least one light source (604) emitting a modulated light radiation (606); and
- at least one sensor (602) according to any one of the preceding claims.

13. The device according to the preceding claim, **characterized in that** it comprises several modulated light sources.

14. The device according to any one of claims 12 or 13, **characterized in that** the modulation frequency of at least one light source is adjustable.

15. A method for detecting a gas, and/or measuring the concentration of a gas, using:
- a sensor (100;300;500) according to any one of claims 1 to 11; or
- a device (600) according to any one of claims 12-14.
